(19) Europäisches Patentamt
European Patent Office
Office européen des brevets

(11) Numéro de publication: **0 402 259 A1**

(12) **DEMANDE DE BREVET EUROPEEN**

(21) Numéro de dépôt: **90401552.6**

(22) Date de dépôt: **07.06.90**

(51) Int. Cl.$^5$: **C07H 15/252, A61K 31/70, C07H 13/04, C07H 13/08, C07D 309/30**

(30) Priorité: **07.06.89 FR 8907544**

(43) Date de publication de la demande: **12.12.90 Bulletin 90/50**

(84) Etats contractants désignés: **AT BE CH DE DK ES FR GB GR IT LI LU NL SE**

(71) Demandeur: **LABORATOIRES HOECHST S.A.**
**TOUR ROUSSEL HOECHST, 1 Terrasse Bellini**
**F-92800 Puteaux(FR)**

(72) Inventeur: **Monneret, Claude**
**9 rue Lamoricière**
**F-75012 Paris(FR)**
Inventeur: **Florent, Jean-Claude**
**23 rue des Causses**
**F-91940 Les Ulis(FR)**
Inventeur: **Gaudel, Gilbert**
**155 Boulevard Vincent Auriol**
**F-75013 Paris(FR)**

(74) Mandataire: **Orès, Bernard et al**
**Cabinet ORES 6, Avenue de Messine**
**F-75008 Paris(FR)**

(54) **Nouvelles anthracyclines, leur procédé de préparation ainsi que médicaments les contenant.**

(57) Ces anthracyclines sont représentées par la formule I ci-après :

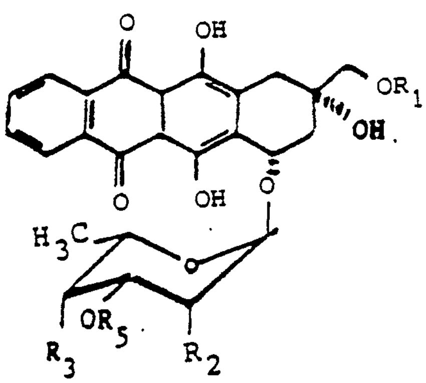

(I)

dans laquelle
$R_1$ représente un atome d'hydrogène, un groupe $COR_4$, dans lequel $R_4$ représente un groupe alkyle en $C_1$-$C_8$ ;
$R_2$ représente un atome d'hydrogène ou un atome d'iode ;
$R_3$ représente un groupe azide, un groupe morpholino, une amine primaire, une amine secondaire $NHR_6$ dans laquelle $R_6$ représente un groupe $COCF_3$, un groupe $COCH_3$, un groupe phtalimido, une amine tertiaire $NR_7R_7$, dans laquelle $R_7$ est un benzyle, un méthyle, un aminonitrile ;
$R_5$ représente un atome d'hydrogène, un groupe acyle en $C_2$-$C_4$ éventuellement substitué, un groupe benzyle éventuellement substitué.

EP 0 402 259 A1

# NOUVELLES ANTHRACYCLINES, LEUR PROCEDE DE PREPARATION AINSI QUE MEDICAMENTS LES CONTENANT.

La présente invention est relative à de nouvelles anthracyclines, à leur procédé de préparation ainsi qu'à des médicaments les contenant.

Les anthracyclines représentées par la formule générale ci-après (A) :

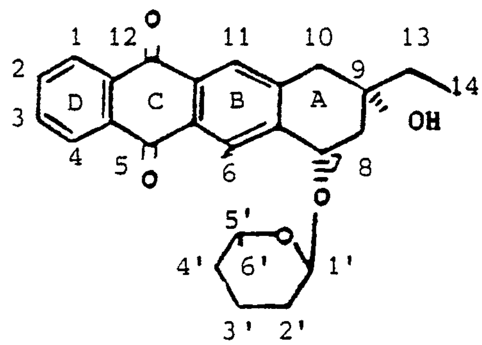

(A)

constituent une famille importante d'antibiotiques et d'antitumoraux. Certains membres de cette famille (comme par exemple la daunorubicine et la doxorubicine) sont utilisés en clinique avec succès, pour le traitement de diverses formes de cancer (leucémies aiguës, cancers du sein, cancers des voies urinaires, maladies de HODGKIN, etc...).

Leur remarquable activité cytotoxique est néanmoins limitée par des effets secondaires néfastes, et en particulier par une cardiotoxicité importante. Celle-ci liée à la dose d'antibiotique utilisée, nécessite l'interruption irréversible du traitement à partir d'un certain seuil.

De nombreux efforts ont donc été poursuivis au cours de ces dernières années pour améliorer l'index thérapeutique de ces composés : une diminution de la cardiotoxicité apparaît plus importante qu'une augmentation de leur efficacité pondérale.

Les modifications effectuées portent sur l'aglycone et peuvent concerner tous les cycles (A-B-C-D) et/ou sur le sucre.

En ce qui concerne les modifications portant sur l'aglycone, les produits obtenus sont classés généralement en diverses catégories suivant la substitution du cycle B :

. Dérivés dihydroxylés en 6 et en 11 (à cette catégorie appartiennent la daunorubicine -composé II- et la doxorubicine -composé III).

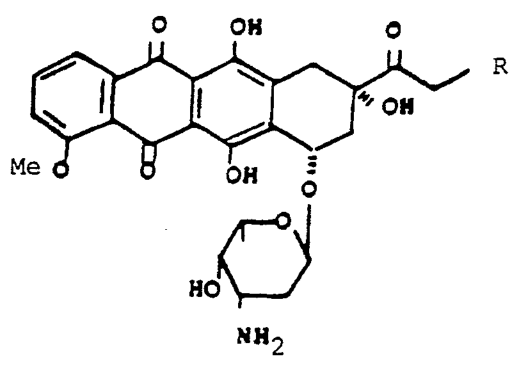

Composé (II) = R = H

Composé (III) = R = OH

. Dérivés monohydroxylés en 6 (série désoxy-11).

. Dérivés monohydroxylés en 11 (série désoxy-6).

Il a été montré précédemment que la substitution de la chaîne latérale de ces glycosides, respectivement $COCH_3$ et $COCH_2OH$, par un groupement $CH_2OH$ conduit à de nouveaux glycosides antitumoraux qui présentent une activité antitumorale similaire à celle des produits de l'Art antérieur mais ils ne présentent pas de résistance croisée avec la doxorubicine.

On peut citer notamment le Brevet européen 44 954 au nom de F. HOFFMANN-LAROCHE & CO. et le

Brevet français 84 03634 au nom des LABORATOIRES HOECHST qui décrivent des glycosides comportant de telles anthracyclinones.

En ce qui concerne les modifications portant sur le sucre, de très nombreuses recherches ont été effectuées sur ces molécules pour déterminer la relation entre leur structure et leur activité biologique. Il ressort de ces recherches que des modifications au niveau des sucres aboutissent souvent à des analogues pharmacologiquement plus intéressants que le produit de départ. Ces modifications entraînent, en effet, une diminution de la dose thérapeutiquement efficace, une activité antitumorale plus marquée, des réactions secondaires, notamment la cardiotoxicité, diminuées (cf. notamment les travaux de F. ARCAMONE "Doxorubicine" Academic Press, New York, 1981) sur la daunosamine, sucre des daunorubicine et doxorubicine.

Parmi les modifications proposées figure, entre autres, la désoxygénation du C-4′ du sucre (cf. Brevet Européen 0 048 967 de FARMITALIA) ; selon les Inventeurs de ce Brevet, le produit 3 représenté sur la figure B ci-après présente une activité anti-tumorale très intéressante.

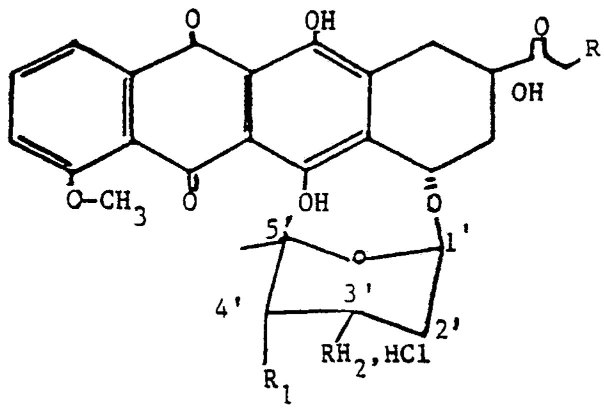

(B)

B1 $R_1$ = OH ; R = H (daunorubicine)
B2 $R_1$ = R = OH (doxorubicine)
B3 $R_1$ = R = H (Brevet Européen 0 048 967)

L'inversion de la configuration du sucre au niveau des carbones en 3′ et/ou 4′ -voir figure C ci- après- :

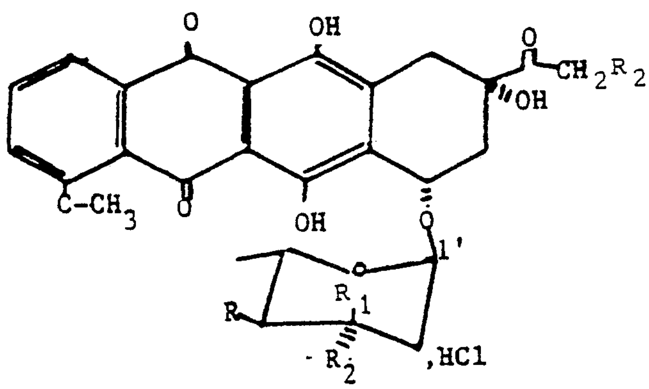

(C)

C4 R = OH ; $R_1$ = H ; $R_2$ = $NH_2$
C5 R = $R_2$ = H ; $R_1$ = $NH_2$

contribue également à préparer des dérivés pharmacologiquement très intéressants. Le glycoside C4 a une forte activité antitumorale et le produit désoxygéné C5 a fait l'objet d'un Brevet (Carlo ERBA, Jpn.Kokai Tokkyo Koho JP 57-142-981), FARMITALIA, BE 891,837).

Les sucres des formules B et C comportent une fonction amine en C-3′ ; la présence d'une telle fonction amine primaire ou secondaire en C-3′ (la daunosamine par exemple) semble indispensable pour l'activité antitumorale de ces produits, en particulier en raison de son rôle dans le processus d'intercalation dans l'ADN (G.L. TONG et al., J. Med. Chem. 1979, 22, 912 et A. DIMARCO et al., Arzneim Forch, 1975, 25, 368).

Le Brevet américain 4 684 629, au nom de FARMITALIA Carlo ERBA S.p.A, décrit des anthracyclines

dérivées de la daunorubicine ou de la doxorubicine (voir formule B ci-dessus) comportant un sucre aminé en 4'.

Cependant, ces anthracyclines ont l'inconvénient de présenter une résistance croisée avec la doxorubicine.

La Demanderesse, poursuivant l'étude entreprise déjà depuis plusieurs années (cf. notamment les Brevets français 2 551 057, 2 560 876, 2 565 982, 2 584 406 et 2 591 599) a mis au point de nouvelles anthracyclines qui sont d'une part des agents cytotoxiques et antitumoraux puissants, tout en ne présentant pas une cardiotoxicité accrue par rapport aux produits de l'Art antérieur et d'autre part, dont le procédé de préparation est facile à mettre en oeuvre et peu coûteux, car présentant un bon rendement.

La présente invention a pour objet des anthracyclines, représentées par la formule I ci-après :

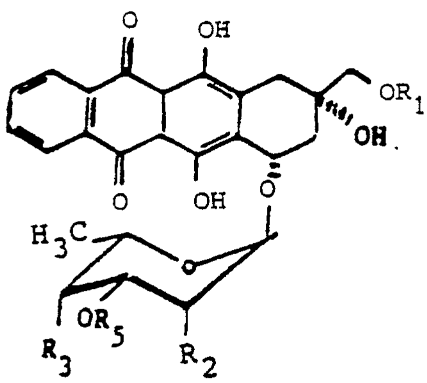

(I)

dans laquelle

**$R_1$** représente un atome d'hydrogène, un groupe COR4, dans lequel **$R_4$** représente un groupe alkyle en $C_1$-$C_8$ ;

**$R_2$** représente un atome d'hydrogène ou un atome d'iode ;

**$R_3$** représente un groupe azide, un groupe morpholino, une amine primaire, une amine secondaire $NHR_6$ dans laquelle **$R_6$** représente un groupe $COCF_3$, un groupe $COCH_3$, un groupe phtalimido, une amine tertiaire $NR_7R_7$, dans laquelle $R_7$ est un benzyle, un méthyle, un aminonitrile ;

**$R_5$** représente un atome d'hydrogène, un groupe acyle en $C_2$-$C_4$ éventuellement substitué, un groupe benzyle éventuellement substitué.

Les anthracyclines conformes à la présente invention ont l'avantage de présenter un gain de puissance d'activité sans augmenter leur toxicité. Elles ont égale ment l'avantage de ne pas présenter de résistance croisée avec la doxorubicine.

La présente invention a également pour objet un procédé de préparation des anthracyclines conformes à l'invention, caractérisé en ce qu'il comprend les étapes suivantes :

(1) glycosidation de l'aglycone de formule 1

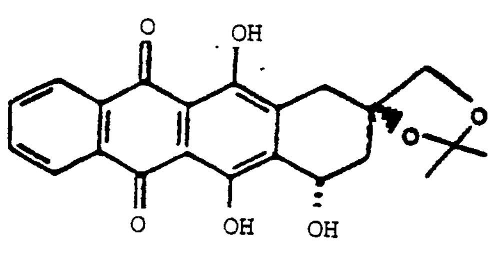

(1)

avec un sucre de formule 1'

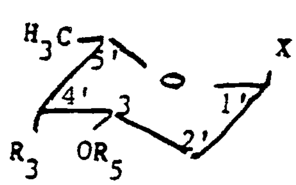

(1')

dans laquelle
X représente un atome de brome ou une double liaison $C1'$-$C2'$ ;
$R_3$ et $R_5$ ont la même signification que ci-dessus ;

(2) hydrolyse sélective du cycle acétalique ;

(3) puis éventuellement saponification du groupe ester en $C\text{-}3'$, lorsque $R_5$ n'est pas un atome d'hydrogène ; et

(4) éventuellement une alkylation de la fonction amine obtenue.

Conformément à l'invention, lorsque $R_3$ est un groupement amine secondaire, le procédé comprend en outre une étape de déprotection de la fonction amine secondaire.

Selon un mode de mise en oeuvre avantageux de ce procédé lorsque X est un atome de brome, la glycosidation est réalisée suivant la réaction de KOENIGS-KNORR selon le schéma I ci-après :

**Schéma I**

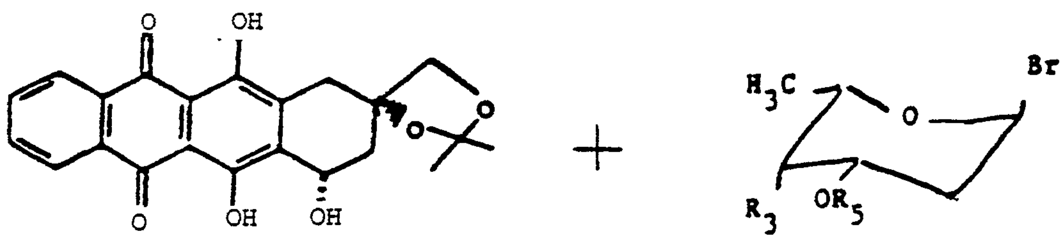

I

1'

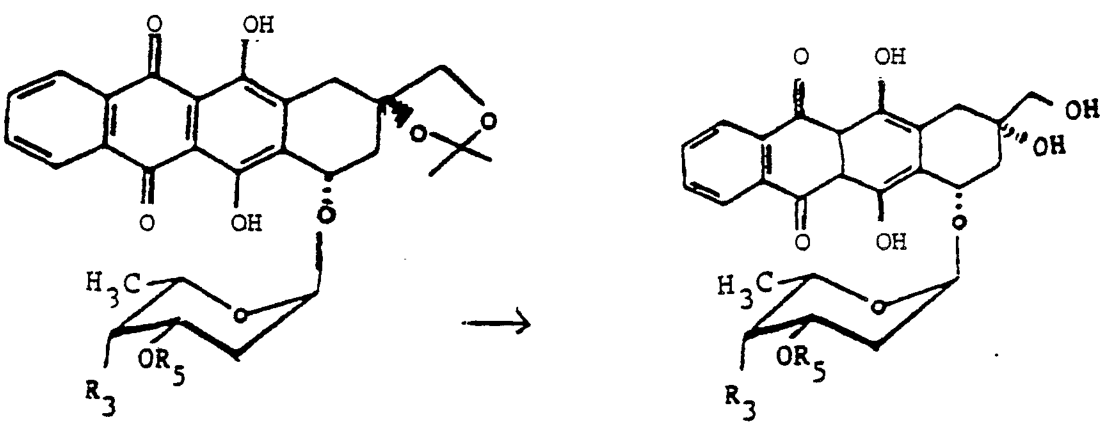

4'

5'

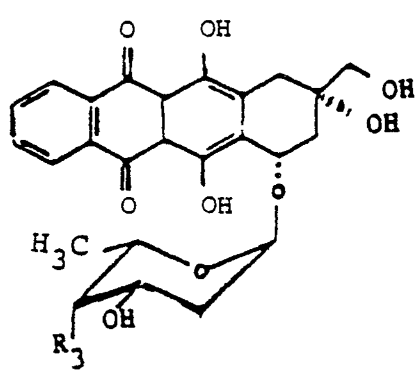

6'

L'aglycone de formule 1 est celle décrite dans la Demande de Brevet au nom de HOECHST n° 2 560 876.

Selon une disposition avantageuse de ce mode de mise en oeuvre, l'aglycone 1 est transformée en anthracycline par glycosidation à l'aide du sucre de formule 3, conformément au Schéma II ci-après :

Schéma II

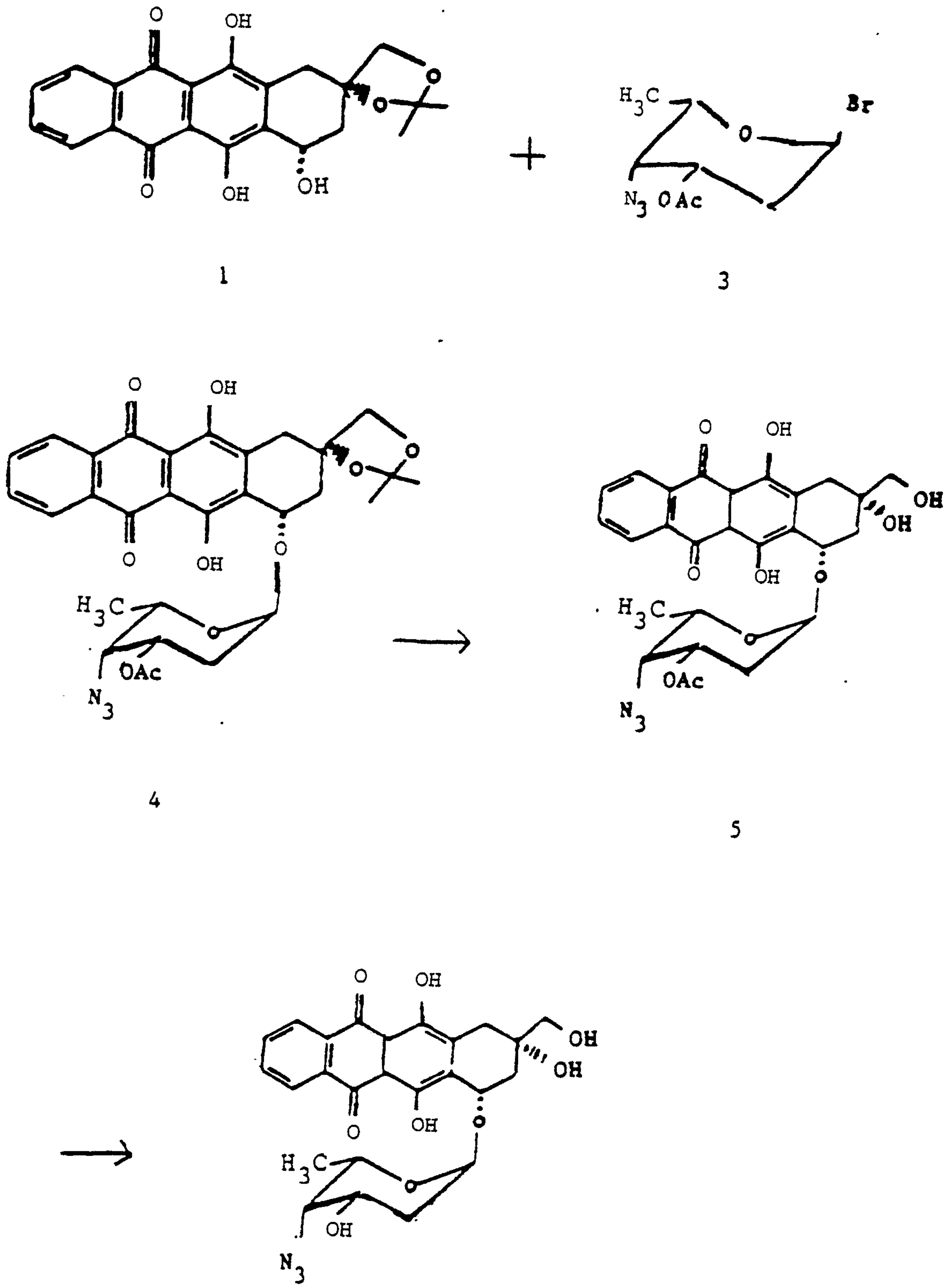

1

3

4

5

6

L'on obtient alors la (S)-cis-1-0-(4-azido-2,4,6-tridesoxy-α-L-lyxo-hexopyranosyl)-1,2,3,4,6,11-hexahydro-1,3,5,12-tétrahydroxy-3-hydroxyméthyl-naphtacène dione de formule 6.

Le bromure de l'azido-sucre 3 est obtenu à partir de l'O-acétyl-1 acétyl-3-azido-4-tridésoxy-2,4,6-lyxo-hexopyranose de formule 2 ci-après :

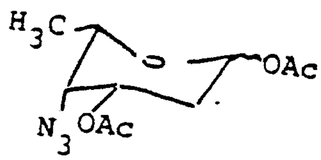

(2)

par bromation, lui-même obtenu à partir du méthyl azido-4-O-benzoyl-3-tridésoxy-2,4,6,L-lyxo-hexopyranose décrit par MARTIN et al., Carbohydr. Res., 1987, 166, 59. Typiquement 2 s'obtient en traitant ce méthyl glycoside décrit dans la littérature par un mélange d'acide sulfurique dans l'anhydride acétique et d'acide acétique. Le dérivé halogéné 3 est ensuite obtenu par action de HBr sur 2 en solution dans du benzène.

Selon une autre disposition avantageux de ce mode de réalisation, l'aglycone 1 est transformée en anthracycline par glycosidation à l'aide du sucre de formule 8, conformément au schéma III ci-après :

**Schéma III**

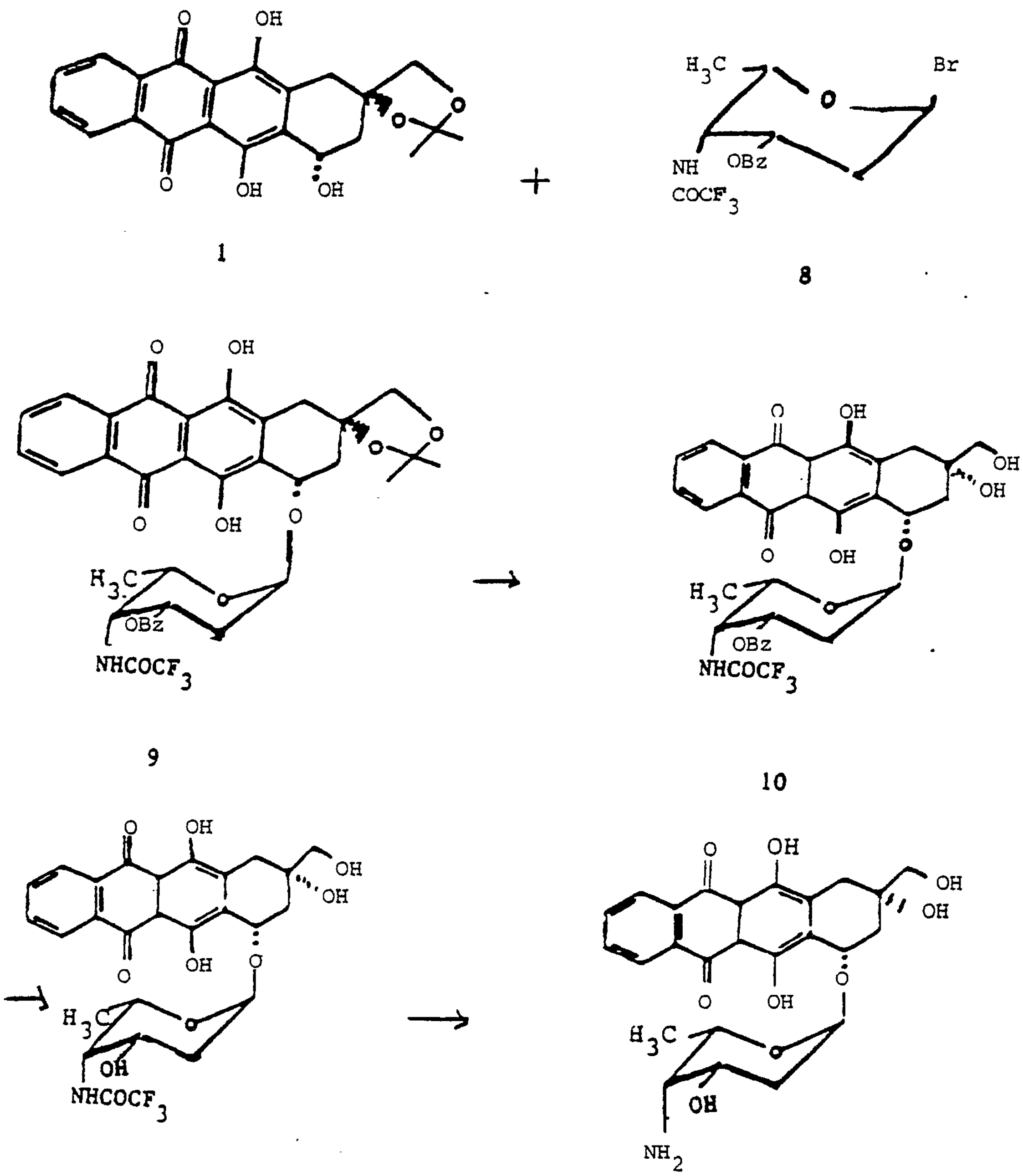

1 8 9 10 11 12

L'on obtient alors la (S)-cis-1-O-(4-amino-2,4,6-trideoxy-α-L-lyxo-hexopyranosyl)-1,2,3,4,6,11-hexahydro-1,3,5,12-tétrahydroxy-3-hydroxyméthyl-6,11 naphtacène dione de formule 12.

Le bromo-sucre 8 résulte de l'action de HBr sur le dérivé correspondant acétylé de formule 7, ci-après :

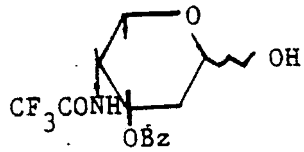

(7) ,

décrit par A. MARTIN et al. (Carbohydr. Res., 1987, 166, 199).

Selon une modalité avantageuse de cette disposition par alkylation du produit 12 obtenu ci-dessus, on obtient le composé de formule 14 ci-après :

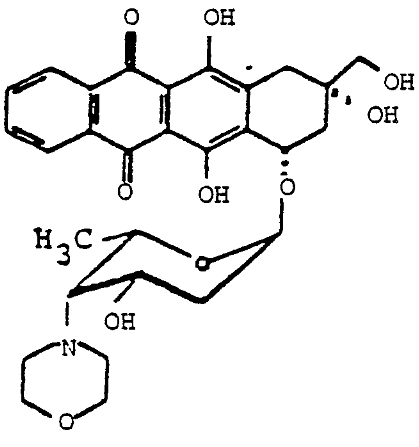

(14)

Selon un autre mode de réalisation avantageux du procédé conforme à l'invention, lorsque X est une double liaison, la glycosidation est réalisée en présence de N-iodosuccinimide selon le schéma IV ci-après :

**Schéma IV**

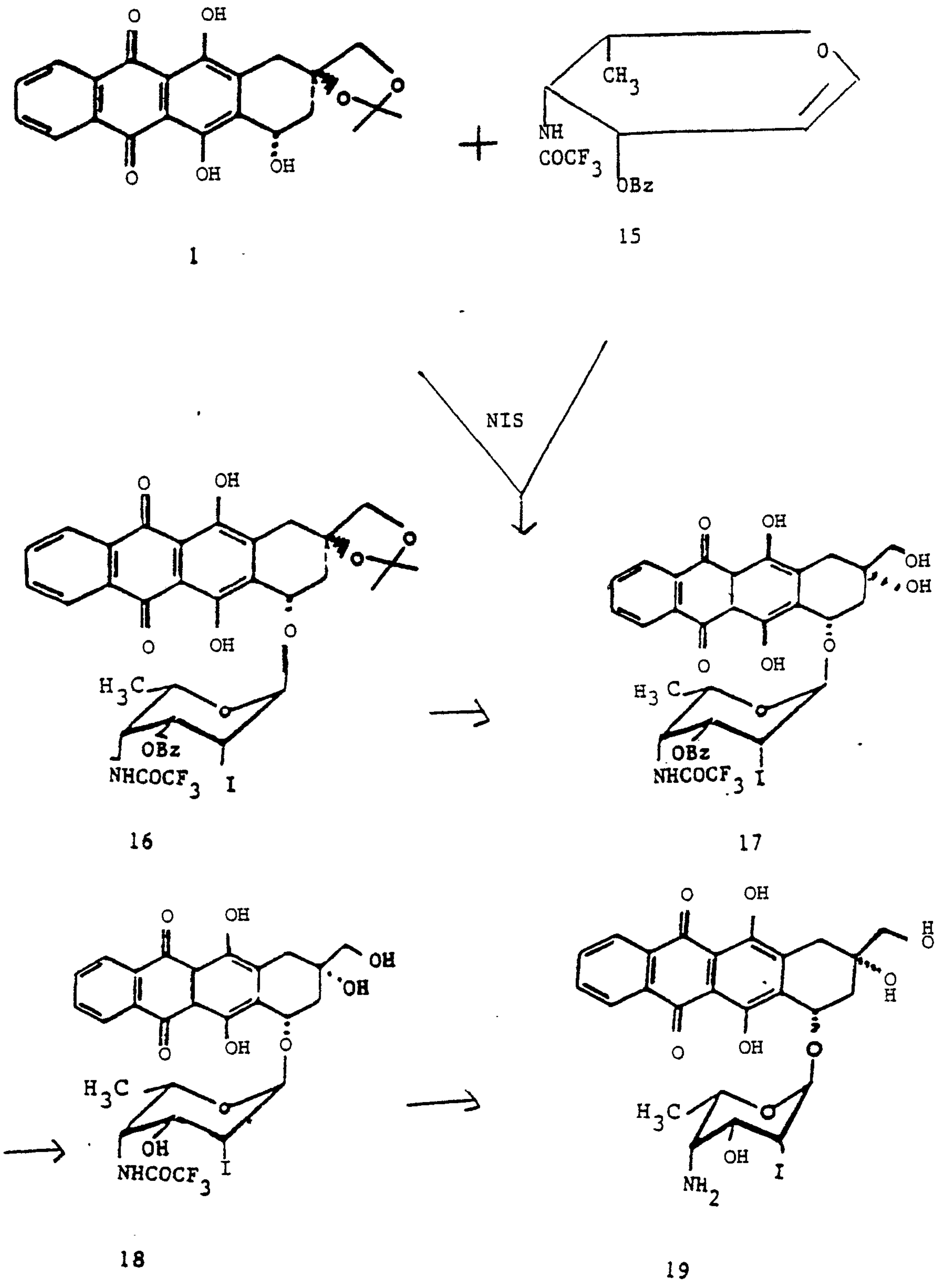


1 15 16 17 18 19

Le glycal de formule 15 est également préparé à partir du produit de formule 7 tel que défini ci-dessus, par hydrolyse acide en présence d'un acide minéral faible, et agitation dans la pyridine en présence de chlorure de méthane sulfonyle et de triéthylamine.

Outre les dispositions que précèdent, l'invention comprend encore d'autres dispositions qui ressortiront de la description qui va suivre.

L'invention vise plus particulièrement les nouvelles anthracylines et leur procédé de préparation, ainsi que les compositions, en particulier les compositions à visée thérapeutique les contenant, pour leur utilisation en chimiothérapie anti-cancéreuse.

Ces nouvelles anthracyclines ont des propriétés cytotoxiques et antitumorales remarquables, comme précisé dans les tests pharmacologiques ci-après.

Parmi les différentes anthracyclines conformes à la présente invention, présentant des résultats pharmacologiques très intéressants, il y a lieu de citer notamment :

. la (S)-cis-1-0-(4-azido-2,4,6-trideoxy-α-L-lyxo-hexopyranosyl)-1,2,3,4,6,11-hexahydro-1,3,5,12-tétrahydroxy-3-hydroxyméthyl-6-11-naphtacène dione de formule 6 ;

. la (S)-cis-1-O-(4-amino-2,4,6-trideoxy-α-L-lyxo-hexopyranosyl)-1,2,3,4,6,11-hexahydro-1,3,5,12-tétrahydroxy-3-hydroxyméthyl-6,11 naphtacène dione de formule 12 ;

. le chlorhydrate de (S)-cis-1-O-(4-amino-2,4,6-trideoxy-α-L-lyxo-hexopyranosyl)-1,2,3,4,6,11-hexahydro-1,3,5,12-tétrahydroxy-3-hydroxyméthyl-6,11-naphtacène dione de formule 13 ;

. la (S)-cis-1-O-(3-morpholino-2,4,6-tridésoxy-α-L-lyxohexopyranosyl)-1,2,3,4,6,11-hexahydro-1,3,5,12-tétrahydroxy-3-hydroxyméthyl-6,11-naphtacène dione de formule 14 ;

. la (S)-cis-1-O-(4-amino-2,4,6-tridésoxy-2-iodo-α-L-talo-hexopyranosyl)-1,2,3,4,6,11-hexahydro-1,3,5,12-tétrahydroxy-3-hydroxyméthyl-6,11-naphtacène dione de formule 19.

L'invention sera mieux comprise à l'aide du complément de description qui va suivre, qui se réfère à des exemples de mise en oeuvre du procédé objet de la présente invention ainsi qu'à des exemples mettant en évidence les propriétés thérapeutiques desdits glycosides.

**Exemple 1 : (S)-cis-1-O-(3-O-acétyl-4-azido-2,4,6-tridésoxy-α-L-lyxo-hexopyranosyl)-1,2,3,4,6,11-hexahydro-1,3,5,12-tétrahydroxy-3-hydroxyméthyl-3,13-isopropylidène-6,11-naphtacène dione, de formule 4.**

A une solution d'aglycone 1 (140 mg, 0,35 mmol) dans du dichlorométhane (150 ml) on ajoute de l'oxyde jaune de mercure (0,87 g, 4mmol), du bromure mercurique (0,23 g, 0,68 mmol) et du tamis moléculaire pulvérisé 4A (1,5 g). Le bromo-sucre 3 préparé extemporanément à partir de 2 (184 mg soit 0,71 mmol avec barbottage en solution benzénique avec un courant de HBr durant 5 min puis coévaporation deux fois avec du benzène anhydre) est ajouté en dernier. On agite à température ambiante durant 18 h puis on extrait par du dichlorométhane de façon habituelle. Ceci fournit 250 mg de produit brut qui est chromatographié sur gel de silice avec un mélange d'hexane-EtOAC (4:1) comme éluant. On isole 200 mg (soit 96 %) de 4 pur. Une cristallisation de l'acétate d'éthyle donne :

F 143-145° (déc) ;

$[\alpha]_D^{20}$ + 148° (c 1, chloroforme) ;

IR (chloroforme) : 2100 (azide), 1725 (ester) et 1680 $cm^{-1}$ (quinone chélatée) ;

RMN $^1H$ : δ8,25 (m) et 7,75 (m) (4 H aromatiques), 5,50 (m.$W_{1/2}$ 7 Hz, H-1), 5,20 (m, J = 10 Hz, J′ = 4 Hz, J″ = 3 Hz, H-3′), 5,00 (m, J = J′ = 3 Hz, H-1′), 4,27 (q, J = 6,5 Hz, J′<0,5 Hz, H-5′), 3,89 (d) et 3,82 (d) (AB, J = 9 Hz, $CH_2$-13), 3,78 (m, J = 3 Hz, J′<0,5 Hz, H-4′), 3,24 (d) et 2,82 (d) (AB, J = 18 Hz, $CH_2$-4), 2,03 (s. OAc), 1,46 (s. 2 Me,* isopropylidène), 1,32 (d, J = 6,5 Hz, $CH_3$-6′) ;

DCI/$NH_3$ : m/z 611 (M + $NH_4^+$), 414, 396, 233, 215, 155, 132 ;

Rf : Hexane-EtOAC (4:1) : 0,30.

**Analyse** : Calculé pour $C_{30}H_{31}O_{10}N_3$ (593.57) : C, 60,70 ; H, 5,26, N, 7,08. Trouvé : C, 60,75 ; H, 5,35, N. 7,10.

**Exemple 2 : (S)-cis-1-O-(3-O-acétyl-4-azido-2,4,6-tridesoxy-α-L-lyxo-hexopyranosyl)-1,2,3,4,6,11-hexahydro-1,3, 5,12-tétrahydroxy-3-hydroxyméthyl-6,11-naphtacène dione, de formule 5.**

A une solution de 4 (150 mg) dans un mélange d'acétone (3 ml) et méthanol (3 ml), on ajoute 24 ml d'acide acétique puis 3 ml d'eau et la solution est chauffée et agitée durant une nuit à 50° C. Une extraction par EtOAC donne 140 mg de produit brut qui est chromatographié sur gel de silice avec un mélange de dichlorométhane-MeOH (95:5) comme éluant. On isole 80 mg (57 %) de 5 pur qui cristallise de l'acétate d'éthyle :

F 210° C ;

$[\alpha]_D^{20}$ + 136° (c, 0,1, chloroforme) ;

IR : cf. Exemple 1

RMN $^1H$ : δ 13,42 (s) et 13,17 (s) (OH phénols), 8,25 (m) et 7,76 (m) (4 H aromatiques), 5,50 (m, $W_{1/2}$ ≈ 7Hz, H-1) 5,20 (m, $W_{1/2}$ ≈ 8 Hz, H-1′), 5,07 (m, J = 12 Hz, J′ = J″ = 3 Hz, H-3′), 4,20 (q, J = 6,5 Hz, J′>0,5 Hz, H-5′), 3,71 (d) et 3,50 (d) (AB, J = 11 Hz, $CH_2$-13), 3,20 (d) et 2,55 (d) (AB, J = 18 Hz, $CH_2$-4), 2,02 (s, OAc), 1,36 (d, J = 6,5 Hz, $CH_3$-6′), 2,15-1,80 (m, 4H, $CH_2$-2 et $CH_2$-2′) ;
DCI/$NH_3$ : m/z 571 (M + $NH_4^+$ ), 356, 233, 215 (pic de base) et 138 ;
**Analyse** : Calculé pour $C_{27}H_{27}N_3O_{10}$ (553.51) : C, 58,58 ; H, 4,92 ; N, 7,59. Trouvé : C, 58,62, H, 5,04 ; N, 7,65.

**Exemple 3 : (S)-cis-1-O-(4-azido-2,4,6-trideoxy-α-L-lyxo-hexopyranosyl-1,2,3,4,6,11-hexahydro-1,3,5,12-tétrahydroxy-3-hydroxyméthyl-6-11-napthacène dione, de formule 6.**

L'anthracycline 6 est préparée par glycosidation de l'aglycone (Brevet Hoechst n° 2 560 876) avec le bromure de l'acétyl-3-azido-4-tridésoxy-2,4,6-hexopyranose 3 puis hydrolyse sélective du cycle acétalique de l'anthracycline 4 et enfin saponification du groupe ester en C-3′ de l'anthracycline 5. Typiquement, la condensation de 1 avec 3, dans le dichlorométhane anhydre est réalisée avec 96 % de rendement, en présence d'oxyde jaune de mercure, bromure mercurique et tamis moléculaire. L'hydrolyse du cycle acétalique de 4, est réalisée dans un mélange acide acétone-méthanol par addition d'acide acétique et eau. Enfin la saponification de l'ester en C-3′ s'obtient en traitant 5 par la soude aqueuse à 0° C.

A une solution de 5 (60 mg) dans du dichlorométhane (25 ml) et du méthanol (3 ml) refroidie à 0° C, on ajoute 1 ml de soude aqueuse 0,25N. L'agitation est maintenue à 0° C durant 3 heures puis le mélange est neutralisé par addition goutte à goutte d'AcOH. Après virage de la coloration au rouge, on ajoute une solution saturée de NaCl (= 20 ml) puis on extrait par de l'acétate d'éthyle. Ceci permet d'isoler 60 mg de 6 sous forme de résidu cristallin. Recristallisé du méthanol, 6 donne :

F 241-242° C ;
[α] $^{20}_{D}$ 83° (c 0.018, MeOH) ;
IR (chloroforme) : 2100 (azide) ;
RMN $^1H$: δ 13,40 (s) et 12,40 (s) (0H phénols), 8,22 (m) et 7,74 (m) (4 H aromatiques), 5,45 (m, $W_{H^{1/2}}$ ≈ 7Hz, H-7), 5,17 (dd, J = J′ = 3 Hz, H-1′), 4,13 (q, J = 6,5, J′>0,5 Hz, H- 5′), 3,98 (m, H-3′), 3,65 (d, J = 10) et 3,47 (d, J = 10), (AB, J = 10 Hz, $CH_2$-13), 3,58 (large s, H-4′), 3,16 (d) et 2,53 (d) (AB, J = 18, $CH_2$-4), 2,10-1,60 (m, $CH_2$-2′ et $CH_2$-2), 1,38 (d, J = 6,5 Hz, $CH_3$-6′) ;
DCI/$NH_3$ : m/z 529 (M + $NH_4^+$), 374, 356, 338, 321, 275 ;
Rf 0,22 ($CH_2Cl_2$ : MeOH, 95:5, v/v) ;
**Analyse** : Calculé pour $C_{25}H_{25}O_9N_3$ (511,47) C, 58,70 ; H, 4,92. Trouvé : C, 58,52 ; H, 5,18.

**Exemple 4 : Acétyl 4-azido-3-O-acetyl-tridésoxy-2,4,6-L-lyxo-hexopyranoside de formule (2).**

A une solution de méthyl 4-azido-3-O-benzoyl-tridésoxy-2,4,6-L-lyxo-hexopyranoside (900 mg), dans un mélange d'acide acétique (5 ml) et d'anhydride acétique (5 ml), on ajoute 0,5 ml d'une solution à 5 % d'acide sulfurique pur dans de l'anhydride acétique. On agite cette solution, durant 2 h 30 à température ambiante puis on extrait par de l'éther avec lavage par une solution saturée d'hydrogènocarbonate de sodium.

Ceci fournit 800 mg de 2 sous forme cristalline :

F 48-50° C ;
[α] $^{20}_{D}$ ; -69° (c, 0,2, chloroforme) ;
IR (KBr) : 2110 ($N_3$) et 1722, 1073 cm¹ (ester) ;
DCI/$NH_3$ : m/z 275 (M + $NH_4^+$ ), 215 (M-60) ;
RMN $^1H$ : δ 6,09 (d, J = 3 Hz, H-1), 5,27 (m, J = 10 Hz, J′ = 5 Hz, J″ = 5 Hz, H-3), 3,04 (q, J = 6,5, H-5), 3,75 (large s, $W_{H^{1/2}}$ = 6 Hz, H-4), 2,20 (m, J = 14 Hz, J′ = 10 Hz, J″ = 3 Hz, H-2a), 2,09 (s) et 2,04 (s) (2 OAc), 1,88 (m, J = 14 Hz, J′ = 5 Hz, J″<0,5 Hz, H-2e), 1,19 (d, J = 6,5, $CH_3$-6).
**Analyse** : Calculé pour $C_{10}H_{15}O_5N_3$ (257,25) ; C, 46,69 ; H, 5,88 ; N, 16,33. Trouvé : C, 46,75, H, 5,95 ; N, 16,40.

**Exemple 5 : (S)-cis-1-O-(3-O-benzoyl-2,4,6-trideoxy-4-(trifluoroacétamido)-α-L-lyxo-hexopyranosyl)-1,2,3,4,6,11-hexahydro-1,3,5,12-tetrahydroxy-3-hydroxyméthyl-3,13-isopropylidène-6,11-naphtacène-dione de formule 9.**

A une solution d'aglycone 1 (400 mg, 1 mmol) dans du dichlorométhane anhydre (250 ml), on ajoute de l'oxyde jaune de mercure (3,24 g, 15 mmol), du bromure mercurique (1,12 g, 3,11 mmol) et du tamis moléculaire pulvérisé 4 A (4,8 g). On ajoute ensuite le bromo-sucre 8 préparé extemporanément à partir de 7 (1,3 g, 3,34 mmol dans 50 ml de benzène et barbottage d'HBr gazeux durant 5 min, puis coévaporation deux fois avec du benzène anhydre). Le mélange est agité durant 18 h à température ambiante puis filtré et évaporé. Une chromatographie du résidu sur gel de silice en utilisant le mélange toluène-acétone-AcoH (95:5 : 0,2) comme éluant, fournit 700 mg de 9 (soit, 94 %) qui cristallise de l'acétone-hexane :
F 127-128° C ;
$[\alpha]_D^{20}$ + 67,5° (c, 0,04, chloroforme) ;
IR ($CHCl_3$) 3420 (NH), 1725 (ester), 1670 (amide), 1625 et 1590 $cm^{-1}$ (quinone) ;
RMN $^1H$ : δ 13,69 (s) et 12,92 (s) (OH phénols), 8,27 (m) 7,76 (m) et 7,50 (m) (9H aromatiques), 6,52 (d, J=10 Hz, NH), 5,56 (m, $W_{1/2}$ ≈ 7 Hz, H-1), 5,43 (m, J=10 Hz, J′=3 Hz, J″=3 Hz, H-3′), 5,05 (dd, J=J′=4 Hz; H-1′), 4,60 (q, J=6,5, J′<0,5, H-5′), 4,52 (m, H-4′), 3,92 (d) et 3,85 (d) (AB, J=9 Hz, $CH_2$-13), 3,29 (d) et 2,89 (d) (AB, J=18 Hz, $CH_2$-10), 2,33-2,04 (m, 4H, $CH_2$-2 et $CH_2$-2′), 1,50 (s, 6H, $Me_2$ isopropylidène), 1,24 (d, J=6,5 Hz, $CH_3$-6′) ;
DCI/$NH_3$ : m/z 743 ($M+NH_4^+$), 379 (pic de base) ;
Rf : 0,20 (hexane-acétone, 4:1) et 0,47 (toluène-acétone, 90:10).
**Analyse** : Calculé pour $C_{37}H_{34}O_{11}NF_3$ (725,64) : C, 61,23 ; H, 4,72 ; N, 1,93. Trouvé : C, 61,05 ; H, 4,80 ; N, 1,90.

**Exemple 6 : (S)-cis-1-O-(3-O-benzoyl-2,4,6-tridéoxy-4-(trifluoroacétamido)-α-L-lyxo-hexopyranosyl)-1,2,4,6, 11-hexahydro-1,3,5, 12-tetrahydroxy-3-hydroxyméthyl-6,11-napthcène dione de formule 10.**

Une solution de 9 (700 mg) dans un mélange de méthanol (42 ml), AcOH (42 ml) et eau (6 ml) est agité durant 18 h à 45°. Une extraction par de l'acétate d'éthyle suivie d'une chromatographie avec un mélange dichlorométhane-MeOH-AcOH (95:5:0,5) comme éluant fournit 350 mg de produit de départ 9 puis 330 mg de 10. Une seconde opération réalisée à partir des 350 mg de 9 donne 130 mg de 10 soit un total de 460 mg (70 %). Le composé 10 est cristallisé du méthanol :
F 149-152° C ;
$[\alpha]_D^{20}$ 140° (c, 0,062, chloroforme) ;
RMN $^1H$ : δ 13,46 (s) et 13,15 (s) (OH phénols). 8,21 (m), 7,73 (m) et 7,26 (m) (9H aromatiques), 6,49 (d, J=10 Hz, NH), 5,57 (m, $W_{1/2}$ ≈ 7 Hz, H-1), 5,30 (m, H-3′), 5,25 (m, $W_{1/2}$ ≈ 8 Hz, H-1′), 4,47 (m, H-4′), 4,45 (q, J=6,5, J′<0,5, H-5′), 3,71 (d) et 3,49 (d) (AB, J=12 Hz, $CH_2$-13), 3,23 (d) et 2,60 (d) (AB, J=18 Hz, $CH_2$-4), 2,61-2,50 (m) et 2,31-1,76 (m) ($CH_2$-2 et $CH_2$-2′) ;
DCI/$NH_3$ : m/z 703 ($M+NH_4^+$), 365 et 225 (pic de base) ;
Rf : 0,52 (dichlorométhane-MeOH, 95:5).
**Analyse** : Calculé pour $C_{34}H_{30}NO_{11}F_3$ (685,58) : C, 59,56 ; H, 4,41 ; N, 2,04. Trouvé : C, 59,25 ; H, 4,38, N, 2,20.

**Exemple 7 : (S)-cis-1-O-(2,4,6-tridésoxy-4-(trifluoroacétamido)-α-L-hexopyranosyl)-1,2,3,4,6,11-hexahydro-1,3, 5,12-tétrahydroxy-6,11-naphtacène dione de formule 11.**

A une solution de 10 (690 mg) dans un mélange de dichlorométhane (120 ml) et de méthanol (160 ml) re froidi à 0° C, on ajoute 6 ml de soude aqueuse 1N. Après 6 h à 0° C, la solution est neutralisée par addition goutte à goutte d'acide acétique pur. La solution vire au rouge. On extrait par du dichlorométhane après addition d'une solution saturée de NaCl (10-20 ml). Après lavages habituels puis séchage sur $Na_2SO_4$, une évaporation du solvant sous pression réduite fournit 700 mg de produit brut. Une cristallisation du méthanol donne 340 mg de 11 pur cependant que les eaux-mères chromatographiées avec un mélange dichlorométhane-MeOH (95:5) livrent 110 mg de 11 également pur soit un total de 450 mg (70 %) :
F 206° C ;
$[\alpha]_D^{20}$ + 78° (c, 0,05, dioxanne) ;
RMN $^1H$ : δ 13,44 (s) et 13,16 (s) (OH phénols), 8,23 (m) et 7,74 (m) (4 H aromatiques), 6,41 (d, J=9 Hz, NH), 5,50 (d, J=3 Hz, H-1), 5,23 (m, $W_{1/2}$ ≈ 8 Hz, H-1′), 4,41 (q, J=6,5 Hz, J′<0,5 Hz, H-5′), 4,14 (m, H-3′ et H-4′), 3,71 (d) et 3,50 (d) (AB, J=12 Hz, $CH_2$-13), 3,21 (d) et 2,58 (d) (AB, J=18 Hz, $CH_2$-4), 2,50 (d, J=14 Hz) et 1,82 (dd, J=14 Hz, J′=3 Hz, $CH_2$-2), 2,01 (dd, J=14 Hz, J′=4 Hz, H-2′e), 1,62 (m, H-2′a), 1,26 (d, J=6,5 Hz, $CH_3$-6′) ;

Rf : 0,30 (dichlorométhane-MeOH, 95:5).
**Analyse** : Calculé pour $C_{27}H_{26}O_{10}NF_3$ (581,49) : C, 55,77 ; H, 4,50 ; N, 2,40. Trouvé : C, 55,88 ; H, 4,60 ; N, 2,37.

**Exemple 8 : (S)-cis-1-O-(4 amino-2,4,6-trideoxy-α-L-lyxo-hexopyranosyl)-1,2,3,4,6,11-hexahydro-1,3,5,12-tétrahydroxy-3-hydroxyméthyl-6,11-naphtacène dione de formule 12.**

La synthèse de l'anthracycline 12, résulte de la condensation de l'aglycone 1 avec le bromure de O-benzoyl-3-trifluoroacétamido-4-tridésoxy-2,4,6-L-lyxo-hexopyranose,de formule 8, de manière à obtenir le produit de formule 9, puis suivie de la déprotection de l'aglycone de 9 qui donne 10, de la saponification de la fonction ester en C-3′ qui fournit le composé 11 et enfin de la déprotection de la fonction amine de 11. Le produit de formule 9 est obtenu avec 94 % de rendement par condensation de 1 avec 8 dans le dichlorométhane anhydre en présence d'oxyde jaune de mercure, de bromure mercurique et de tamis moléculaire. L'hydrolyse du cycle acétalique de 9 par un mélange de méthanol, d'eau et d'acide acétique suivie d'une saponification de 10 par la soude aqueuse d'abord à 0 °C puis à température ambiante fournit respectivement 11 et 12. Cette amino-4 anthracycline est isolée sous forme de chlorhydrate 13 par addition d'une solution méthanolique d'HCl.

Le bromo-sucre 8 résulte de l'action de HBr sur le dérivé correspondant acétylé décrit par A. MARTIN et al., Carbohydr. Res., 1987, 166, 199.

A une solution de 11 (250 mg, 0,43 mmol) dans du tétrahydrofuranne (10 ml), on ajoute 40 ml de soude 0,1 N et on agite à température ambiante durant 5 h. On acidifie ensuite par addition de HCl 5 N jusqu'à pH 4. Après addition de dichlorométhane, on neutralise par addition d'une solution saturée de bicarbonate de sodium.

La phase organique est séparée, lavée par de l'eau puis de l'eau saturée de chlorure de sodium et enfin évaporée sous pression réduite. Le résidu est repris dans un mélange 9:1 de chloroforme et de méthanol (10 ml), refroidi à 0 °C. On acidifie la solution à pH 3 par du méthanol chlorhydrique et on évapore sous pression réduite. Le résidu est dissous dans 1 ml de méthanol auquel on ajoute ensuite de l'éther. Une filtration fournit le chlorhydrate 13,

F 185-190 °C ;
$[\alpha]_D^{20}$ , -67° (c, 0,03, MeOH).
**Analyse** : Calculé pour $C_{25}H_{28}NO_9$ : C, 57,50 ; H, 5,40 ; N, 2,68. Trouvé : C, 57,60, H, 5,38 ; N, 2,74.

**Exemple 9 : (S)-cis-1-O-(4-morpholino-2,4,6-tridésoxy-α-L-lyxo-hexopyranosyl)-1,2,3,4,6,11-hexahydro-1,3,5,12-tétrahydroxy-3-hydroxyméthyl-6,11-naphtacène dione de formule 14.**

La synthèse de dérivés de la fonction amine comme la déamino-3 morpholino-4 anthracycline 14 qui est obtenue par alkylation de 12 en présence de cyanoborohydrure de sodium par le 2,2′-oxabis-(acétaldéhyde). Ce dernier est préparé selon OTEY et al., J. Org. Chem., 1961, 26, 1673, à partir de l'anhydro-1,4-erythritol. L'anhydroerythritol-1,4 (620 mg) en solution dans 10 ml d'eau est agité à température ambiante durant 12 h en présence de périodate de sodium (600 mg). Le mélange est alors neutralisé par addition d'hydrogénocarbonate de sodium puis dilué par de l'acétonitrile. Le filtrat est agité en présence de 12 (20 mg, 0,04 mmol) en solution dans l'acétonitrile auquel on a précédemment ajouté du cyanoborohydrure de sodium (15 mg) dissous dans 5 ml d'acétonitrile-eau (1:1, v/v). Après 15 min, le milieu réactionnel est dilué par une solution aqueuse d'hydrogénocarbonate de sodium puis extrait par de l'acétate d'éthyle. Une chromatographie avec un mélange $CH_2Cl_2$ : MeOH 90:10, fournit 15 mg de 14 :

F 90 °C, (hexane) ;
$[\alpha]_D^{20}$ + 60° (c, 0,03, THF) ;
IR (KBr) ; 3415 (OH), 1625 et 1590 (quinone chélatée) ;
RMN $^1H$ : δ 8,29 (m) et 7,68 (m), (4H aromatiques), 5,83 (large singulet, $W_{H1/2}$ ≈ 7 Hz, H-1), 5,43 (dd, H-1′), 4,62 (q, J = 6,5 Hz, H-5′), 4,45 (m, H-3′), 4,02 (m, $CH_2$-13), 3,70 (large singulet, $CH_2OCH_2$), 3,48 (d) et 3,25 (d) (AB, J = 18 Hz, $CH_2$-4), 3,10-2,90 (m, $CH_2$-N-$CH_2$), 2,60-2,17 (m, $CH_2$-2 et $CH_2$-2′), 1,58 (d, $CH_3$-6′) ;
DCI/$NH_3$ : m/z 556 (M + $H^+$) , 218, 200.
**Analyse** : Calculé pour $C_{29}H_{33}NO_{10}$ (555,28) : C, 62,27 ; H, 5,99 ; N, 2,52. Trouvé : C, 62,75 ; H, 6,08 ; N, 2,61.

## COMPTE-RENDU PHARMACOLOGIQUE CONCERNANT DES GLYCOSIDES SELON L'INVENTION.

### I - Test de prolifération

A - Protocole opératoire (réduction MTT) :

Des cellules tumorales L1210, A549 ou HT29, à une densité de $5.10^3$/ml dans un milieu RPMI sont incubées dans des plaques de microtitration contenant 96 puits pendant 72 heures (37° C. 5 % $CO_2$, 95 % d'humidité relative) avec différentes concentrations de chacune des anthracyclines conformes à l'invention.

Les témoins consistent en cellules tumorales exposées à un milieu de culture. Quatre puits sont préparés pour chaque concentration en anthracycline et pour le témoin. Après 65 heures, 50 µl de MTT (2,5 mg/ml dans du PBS) sont ajoutés.

Le MTT sera réduit en présence de cellules vivantes en un colorant formazan rouge insoluble. Après 7 à 24 heures d'incubation supplémentaire (dépendant des cellules utilisées), le surnageant est enlevé. Le colorant formazan est solubilisé par l'addition de 100 µl de DMSO dans chaque puits, suivie d'une agitation douce.

L'extinction est mesurée pour chaque puits, à 492 nm (photomètre Multiscan 340 CC Fa. Flow).

B - Résultats :

Les résultats sont exprimés comme le rapport de l'extinction après incubation avec les anthracyclines sur l'extinction obtenue avec les témoins. Le coefficient de variation est inférieur à 15 %.

Les résultats sont représentés dans le Tableau I ci-après :

TABLEAU I

| Produits testés | $CI_{50}$ (µg/ml) | | |
|---|---|---|---|
| | TEST AU MTT | | |
| | L1210 | HT 29 | A 549 |
| 6 | 0,13 | 0,46 | 0,24 |
| 12 | 0,021 | 0,03 | 0,24 |
| 11 | 0,015 | 0,026 | 0,011 |
| 13 | 0,009 | 0,01 | 0,009 |
| 14 | 0,15 | 0,13 | 0,054 |
| doxorubicine | 0,02 | non communiqué | |

### II - Effet sur les cellules leucémiques L1210 :

A - Protocole opératoire :

L'essai est réalisé selon la procédure de Hamburger et Salmon, avec les quelques modifications précisées ci-après.

Le milieu utilisé est remplacé par un milieu de McCoy 5A. Le nombre de cellules mises dans les boîtes est réduite à $5.10^2$ cellules/boîte du fait de la croissance rapide des cellules leucémiques L 1210.

Les cellules sont incubées pendant 1 heure à 37° C en présence de différentes concentrations de la substance testée. Puis les cellules sont lavées deux fois à l'aide de McCoy 5A et mises sur plaque d'agarose selon la méthode de Hamburger et Salmon.

Les boîtes sont mises à l'étuve à 37° C sous une atmosphère de 5 % $CO_2$, 20 % $O_2$ et une humidité relative de 95 %, pendant 5 à 7 jours. Après cette période, les colonies de diamètre supérieur à 60 µm sont comptées à l'aide d'un microscope inversé.

B - Résultats :

Les résultats résumés dans le Tableau II, ci-après sont exprimés en pourcentage de colonies formées à partir des cellules L 1210 traitées par rapport aux témoins non traités. Le coefficient de variation, lors de la répétition des expériences, est inférieur à 15 %.

TABLEAU II

| Produits testés | ESSAI SUR CELLULES LEUCEMIQUES |
|---|---|
| | Expérience d'une heure |
| | L1210 |
| 6 | 0,26 |
| 12 | 0,07 |
| 11 | 0,064 |
| 13 | 0,022 |
| 14 | 0,21 |
| doxorubicine | 0,21 |

**III - Essai in vivo. Etude de l'effet du compose 13 :**

A - Protocole opératoire :

- Préparation des cellules L1210 :

Un liquide d'ascite est prélevé dans des conditions aseptiques chez des souris DBA2 (femelle, 18 -20 g) le septième jours après l'implantation.

L'ascite est lavée trois fois avec du PBS, comptée et finalement diluée dans du PBS, de manière à obtenir $10^6$ cellules/0,2 ml.

- Transfert des cellules pour l'entretien de la lignée cellulaire :

$10^6$ cellules dans 0,2 ml de PBS sont injectées dans le peritoine de souris DBA2 pour la propagation de la lignée cellulaire.

Le transfert est effectué une fois par semaine.

- Transfert des lignées cellulaires pour le test :

$10^6$ cellules dans 0,2 ml de PBS sont injectés intrapéritonéalement à des souris BDF1 (femelle 18 -20 g), 6 animaux/groupe sont utilisés pour chaque concentration de substance et pour le contrôle.

B - Evaluation de l'effet :

a) les animaux sont pesés le premier jour et le cinquième jour après l'administration du médicament. Une perte de poids supérieure à 20 % le cinquième jour est utilisée comme indicateur d'effet toxique du produit.

b) à la fin de l'expérience, (mort de tous les animaux ou soixantième jour de l'essai), le temps moyen de survie des animaux, dans tous les groupes ayant plus de 65 % de survivants au cinquième jour, est évalué selon des procédures standard.

A partir du temps de survie moyen des groupes traités ($MST_T$) et des groupes contrôle ($MST_C$), l'effet antitumoral (T/C) est évalué en fonction de la formule suivante :

$$T/C\ \% = \frac{MST_T}{MST_C} \times 100$$

Les valeurs T/C de plus de 125 % sont considérées comme des indicateurs d'un effet antitumoral significatif.

L'effet antitumoral est précisé dans le Tableau III, ci-après :

TABLEAU III

| | | | L1210 |
|---|---|---|---|
| Nom | programme d'administration | dose optimale (mg/kg/Inj.) | T/C % |
| Doxorubicine | 3 i.v. | 8,1 | 148 |
| Composé 13 | 3 i.v. | 3,1 | 133 |

**IV - Xénogreffes :**

TABLEAU IV

| | LXF | A549 | HT29 |
|---|---|---|---|
| Doxorubicine | + + + | + + | + + |
| Composé 13 | + + | + | + |

Le composé 13 présente une activité antitumorale non négligeable s'exerçant à une dose environ trois fois plus faible que celle de la doxorubicine. Par ailleurs des essais réalisés sur xénogreffes (subrenal capsule assay) montrent que ce composé est actif sur certaines tumeurs solides comme celle du poumon (LXF) et à un moindre degré sur la lignée cellulaire A549 et sur un tumeur du colon (HT29).

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1°) Anthracyclines, représentées par la formule I ci-après :

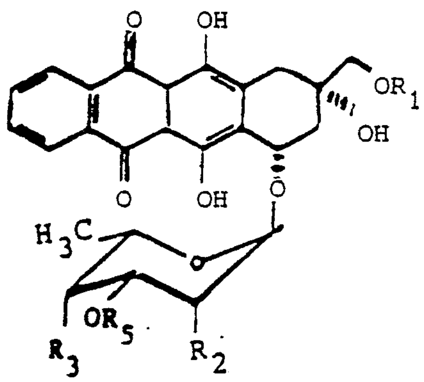

(I)

dans laquelle

**R**$_1$ représente un atome d'hydrogène, un groupe $COR_4$, dans lequel **R**$_4$ représente un groupe alkyle en $C_1$-$C_8$ ;

**R**$_2$ représente un atome d'hydrogène ou un atome d'iode ;

**R**$_3$ représente un groupe azide, un groupe morpholino, une amine primaire, une amine secondaire $NHR_6$ dans laquelle **R**$_6$ représente un groupe $COCF_3$, un groupe $COCH_3$, un groupe phtalimido, une amine tertiaire $NR_7R_4$, dans laquelle **R**$_7$ est un benzyle, un méthyle, un aminonitrile ;

**R**$_5$ représente un atome d'hydrogène, un groupe acyle en $C_2$-$C_4$ éventuellement substitué, un groupe benzyle éventuellement substitué.

2°) Anthracycline selon la revendication 1, constituée par la (S)-cis-1-0-(4-azido-2,4,6-trideoxy-α-L-lyxo-hexo-pyranosyl)-1,2,3,4,6,11-hexahydro-1,3,5,12-tétrahydroxy-3-hydroxyméthyl-6-11-naphtacène dione, de formule 6 ci-après :

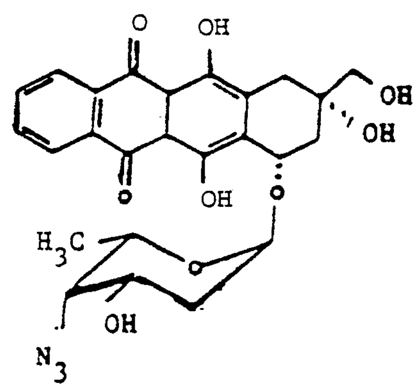

(6)

3°) Anthracycline selon la revendication 1, constituée par la (S)-cis-1-O-(4-amino-2,4,6-trideoxy-α-L-lyxo-hexo-pyranosyl)-1,2,3,4,6,11-hexahydro-1,3,5,12-tétrahydroxy-3-hydroxyméthyl-6,11 naphtacène dione, de formule 12 ci-après :

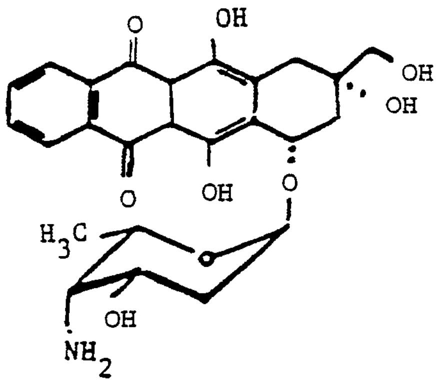

(12)

4°) Anthracycline selon la revendication 1, constituée par le chlorhydrate de (S)-cis-1-O-(4-amino-2,4,6-trideoxy-α-L-lyxo-hexo-pyranosyl)-1,2,3,4,6,11-hexahydro-1,3,5,12-tétrahydroxy-3-hydroxyméthyl-6,11 naphtacène dione, de formule 13 ci-après :

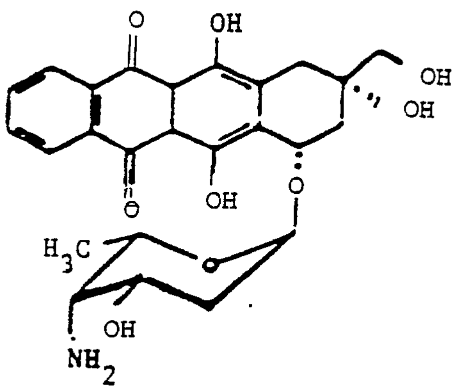

, HCl

(13)

5°) Anthracycline selon la revendication 1, constituée par la (S)-cis-1-O-(4-morpholino-2,4,6-tridésoxy-α-L-lyxo-hexopyranosyl)-1,2,3,4,6,11-hexahydro-1,3, 5,12-tétrahydroxy-3-hydroxyméthyl-6,11-naphtacène dione, de formule 14 ci-après :

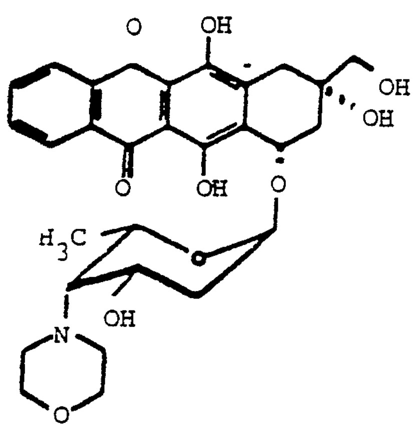

(14)

6°) Anthracycline selon la revendication 1, constituée par la (S)-cis-1-O-(4-amino-2,4,6-tridésoxy-2-iodo-α-L-talo-hexopyranosyl)-1,2,3,4,6,11-hexahydro-1,3,5,12-tétrahydroxy-3-hydroxyméthyl-6,11-naphtacène dione, de formule 19 ci-après :

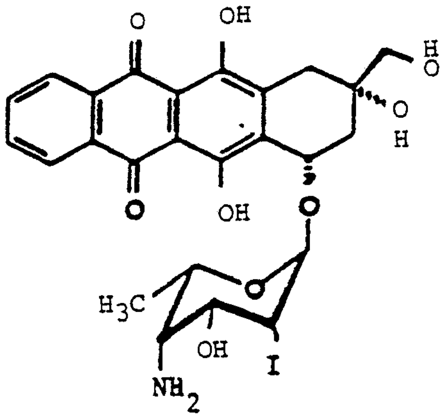

(19)

7°) Procédé de préparation des anthracyclines selon l'une quelconque des revendications 1 à 6, caractérisé par les étapes suivantes :

(1) glycosidation de l'aglycone de formule 1

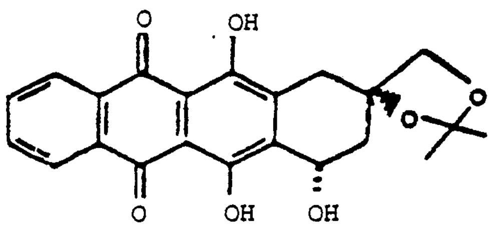

(1)

avec un sucre de formule 1′

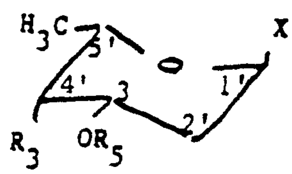

(1′)

dans laquelle

X représente un atome de brome ou une double liaison C1′-C2′ ;

$R_3$ et $R_5$ ont la même signification que ci-dessus ;

(2) hydrolyse sélective du cycle acétalique ;

(3) éventuellement saponification du groupe ester en C-3′, lorsque $R_5$ n'est pas un atome d'hydrogène ;

(4) puis éventuellement une alkylation de la fonction amine en C-4′.

8°) Procédé selon la revendication 7, caractérisé en ce que lorsque $R_3$ est une fonction amine secondaire, il comprend en outre une étape de déprotection de ladite fonction amine secondaire.

9°) Procédé selon la revendication 7, caractérisé en ce que lorsque X est un atome de brome, la glycosidation est réalisée suivant la réaction de KOENIGS-KNORR selon le schéma I ci-après :

**Schéma I**

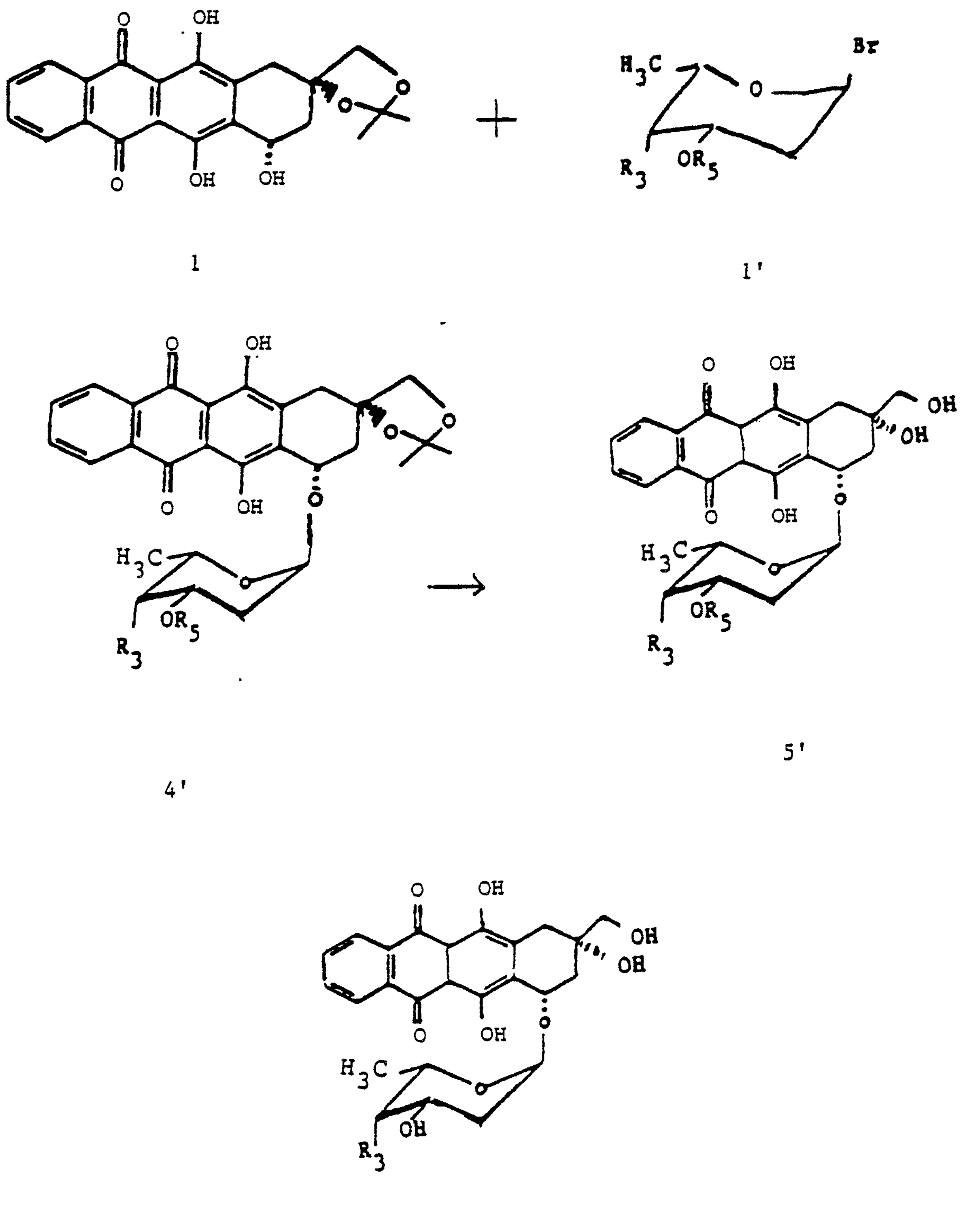

1

1'

4'

5'

6'

10°) Procédé selon l'une quelconque des revendications 7 à 9, caractérisé en ce que l'aglycone 1 est transformée en anthracycline par glycosidation à l'aide du sucre de formule 3, conformément au schéma II ci-après :

Schéma II

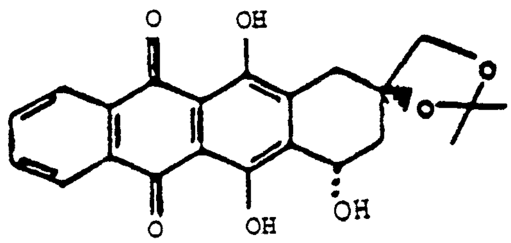

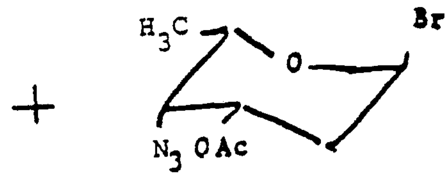

3

1

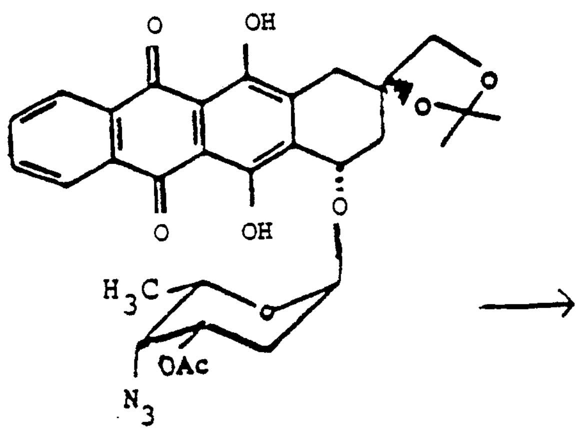

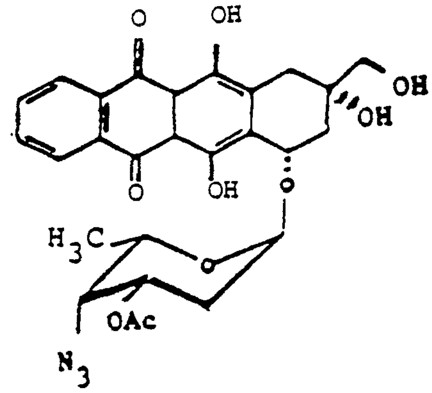

4

5

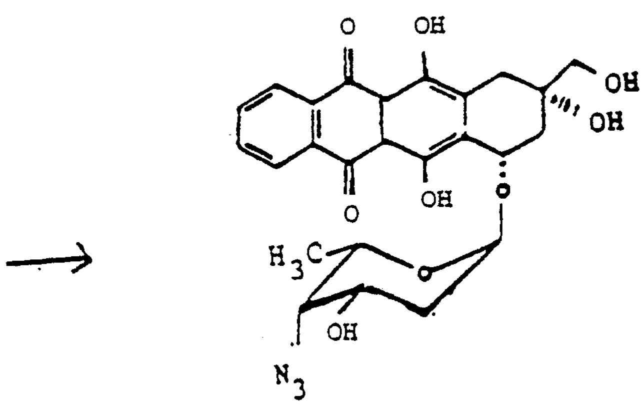

6

11°) Procédé selon l'une quelconque des revendications 7 à 9, caractérisé en ce que l'aglycone 1 est transformée en anthracycline par glycosidation à l'aide d'un sucre de formule 8, conformément au schéma III ci-après :

Schéma III

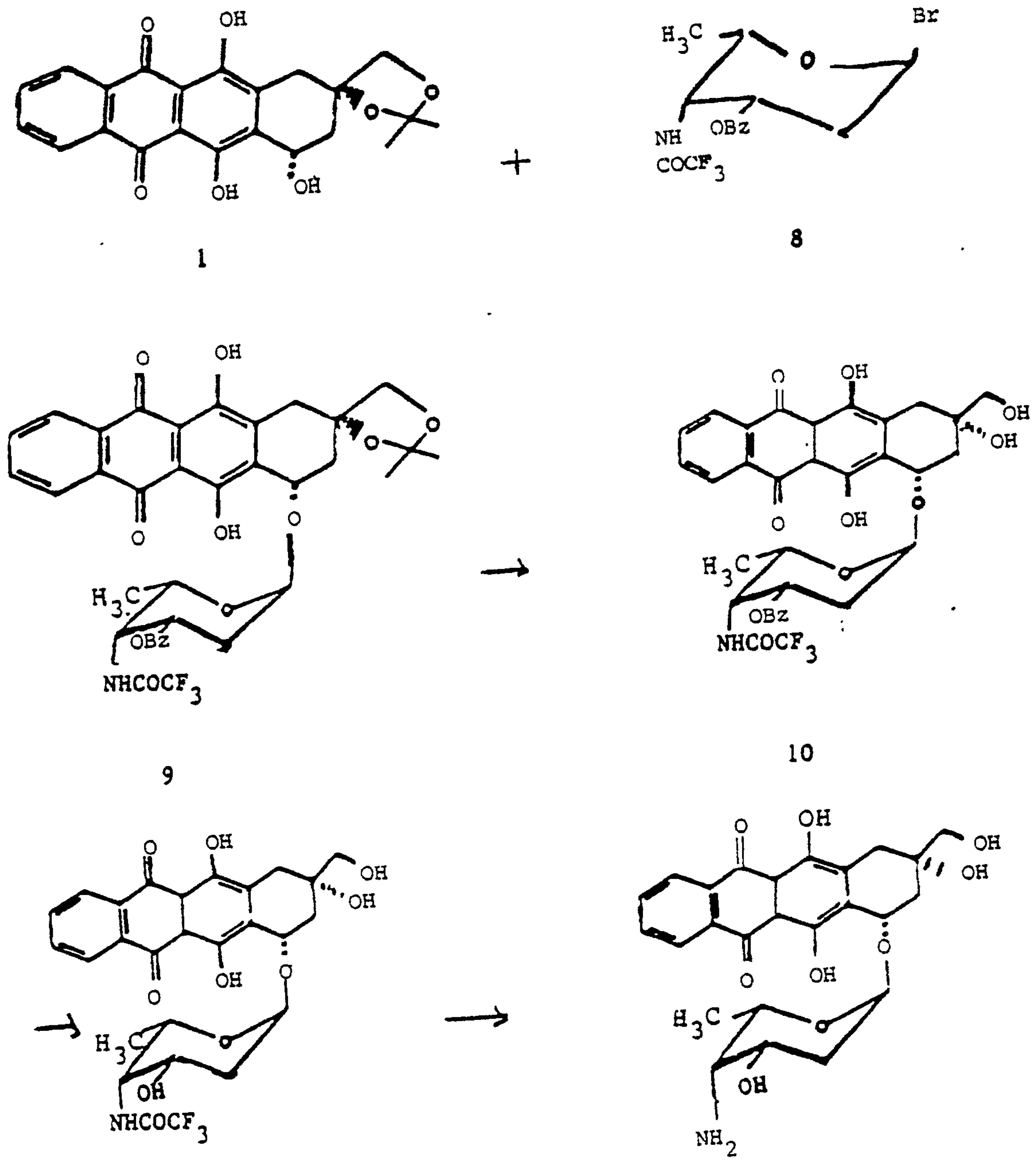

1

8

9

10

11

12

12°) Procédé selon la revendication 7, caractérisé en ce que lorsque X est une double liaison, la glycosidation est réalisée en présence de N-iodosuccinimide selon le schéma IV ci-après :

**Schéma IV**

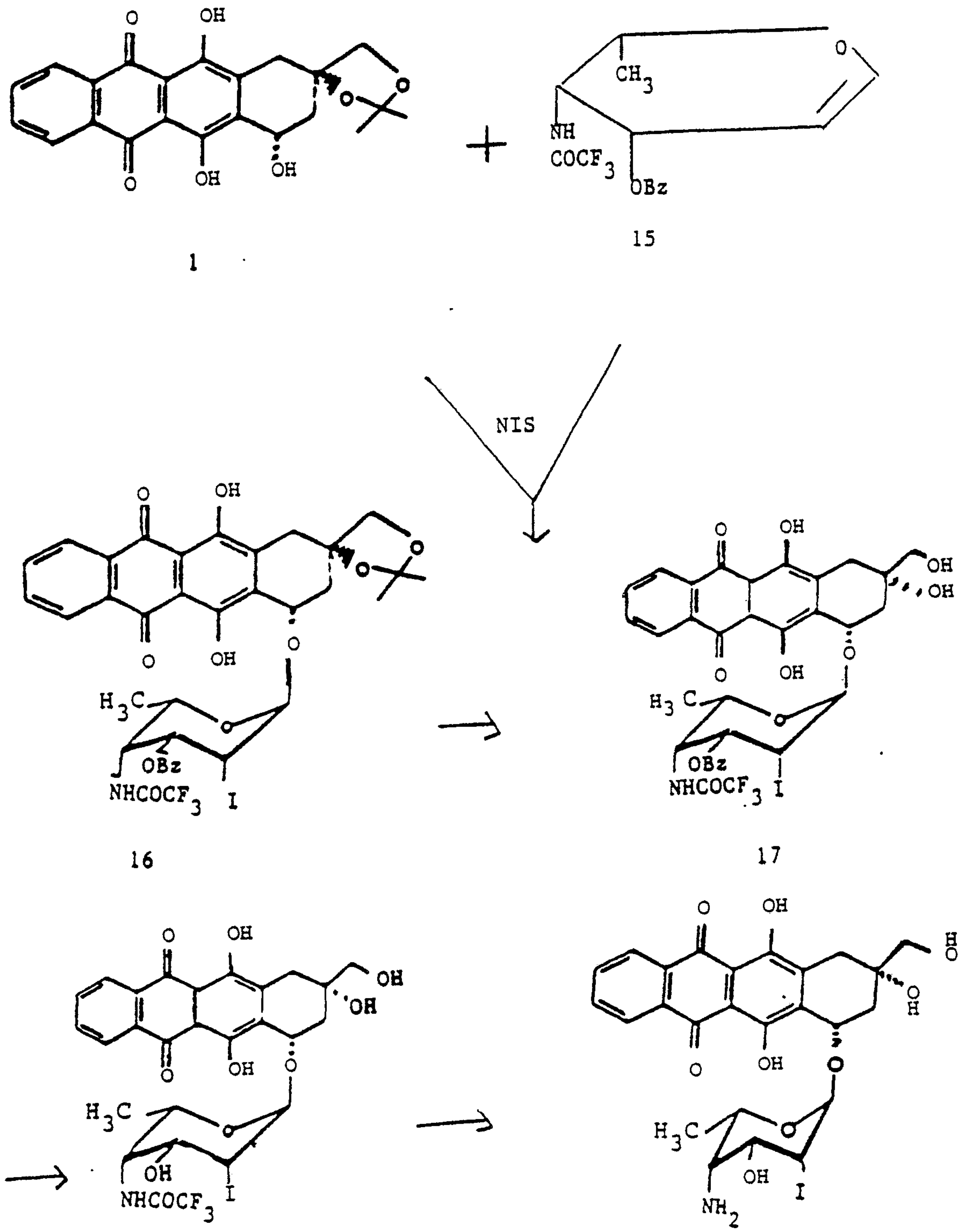


1

15

16

17

18

19

13°) O-Acétyl-1 azido-4-acétyl-3-tridésoxy-2,4,6-L-lyxo-hexopyranose de formule 2, ci-après :

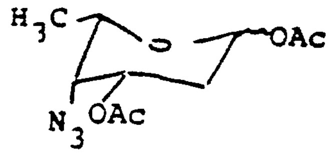

(2),

produit intermédiaire du procédé selon la revendication 10.

14°) Bromure d'azido-4-acétyl-3-tridésoxy-2,4,6-L-lyxo-hexopyranose de formule 3, ci-après :

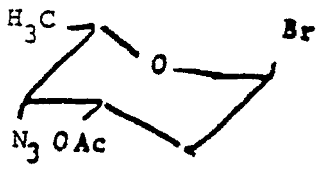

(3),

produit intermédiaire du procédé selon la revendication 10.

15°) Bromure d'O-benzoyl-3 trifluoroacétamido-4-α-L-lyxo-hexopyranose de formule 8, ci-après :

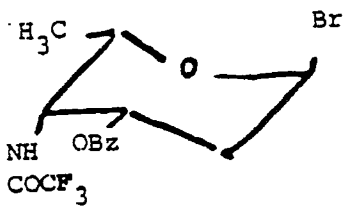

(8)

produit intermédiaire du procédé selon la revendication 11.

16°) Anhydro-1,5-O-benzoyl-3 trifluoroacétamido-4 didésoxy-2,6 D-lyxo hexene-1 itol de formule 15, ci-après :

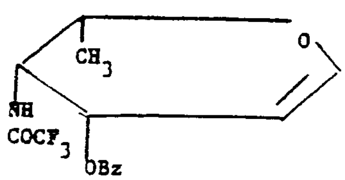

(15)

produit intermédiaire du procédé selon la revendication 12.

17°) (S)-cis-1-O-(3-O-acétyl-4-azido-2,4,6-tridésoxy-α-L-lyxo-hexopyranosyl)-1,2,3,4,6,11-hexahydro-1,3,5,12-tétrahydroxy-3-hydroxyméthyl-3,13-isopropylidène-6,11-naphtacène dione, de formule 4, ci-après :

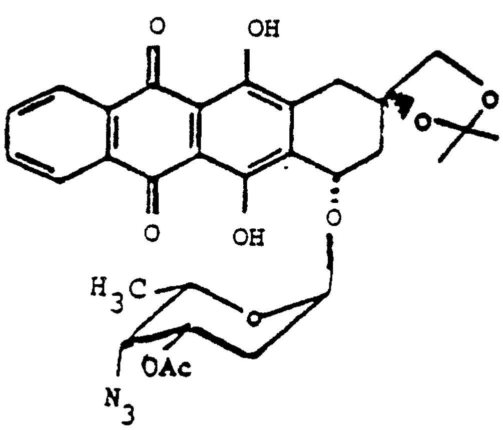

(4),

produit intermédiaire du procédé selon la revendication 10.

18°) (S)-cis-1-O-(3-O-acétyl-4-azido-2,4,6-tridésoxy-α-L-lyxo-hexopyranosyl)-1,2,3,4,6,11-hexahydro-1,3,5,12-tétrahydroxy-3-hydroxyméthyl-6,11-naphtacène dione, de formule 5, ci-après :

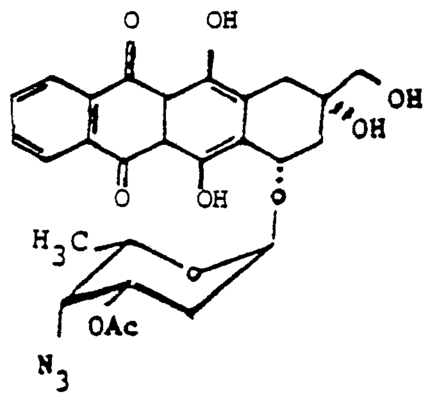

(5),

produit intermédiaire du procédé selon la revendication 10.

19°) (S)-cis-1-O-(3-O-benzoyl-2,4,6-trideoxy-4-(trifluoroacétamido)-α-L-lyxo-hexopyranosyl)-1,2,3,4,6,11-hexahydro-1,3,5,12-tetrahydroxy-3-hydroxy méthyl-3,13-isopropylidène-6,11-naphtacène-dione de formule 9, ci-après :

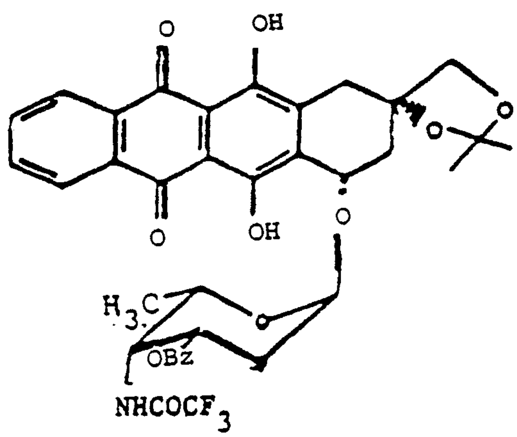

(9)

produit intermédiaire du procédé selon la revendication 11.

20°) (S)-cis-1-O-(3-O-benzoyl-2,4,6-tridéoxy-4-(trifluoroacétamido)-α-L-lyxo-hexopyranosyl)-1,2,4,6,11-hexahydro-1,3,5,12-tetrahydroxy-3-hydroxyméthyl-6,11-napthcène dione de formule 10, ci-après :

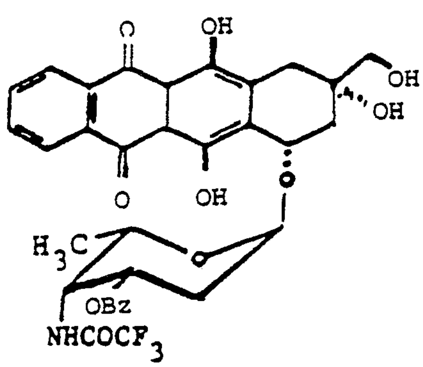

(10)

produit intermédiaire du procédé selon la revendication 11.

21°) (S)-cis-1-O-2,4,6-tridésoxy-4-(trifluoroacétamido)-α-L-hexopyranosyl)-1,2,3,4,6,11-hexahydro-1,3,5,12-tétrahydroxy-6,11-naphtacène dione de formule 11, ci-après :

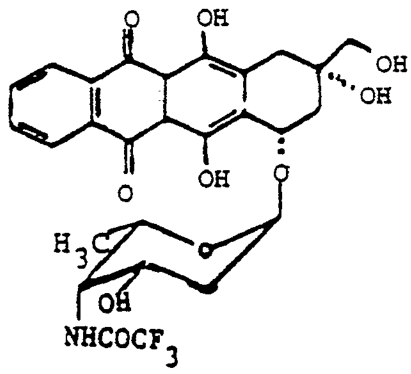

(11)

produit intermédiaire du procédé selon la revendication 11.

22°) Composition pharmaceutique, caractérisée en ce qu'elle contient comme composé actif une anthracycline selon l'une quelconque des revendications 1 à 6.

Office européen des brevets

# RAPPORT DE RECHERCHE EUROPEENNE

Numero de la demande

EP 90 40 1552

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 199 920 (FARMITALIA)<br>* Revendications 1-4,9-11 * | 1,7,22 | C 07 H 15/252<br>A 61 K 31/70<br>C 07 H 13/04<br>C 07 H 13/08<br>C 07 D 309/30 |
| Y | ACTUAL. CHIM. THER., vol. 15, 1988, pages 253-257; C. MONNERET et al.: "Synthèse totale et activité biologique préliminaire d'une nouvelle anthracycline, F 84 0020"<br>* En entier * | 1,7,22 | |

| DOMAINES TECHNIQUES RECHERCHES (Int. Cl.5) |
|---|
| C 07 H 15/00<br>A 61 K 31/00<br>C 07 H 13/00<br>C 07 D 309/00 |

Le présent rapport a été établi pour toutes les revendications

| Lieu de la recherche | Date d'achèvement de la recherche | Examinateur |
|---|---|---|
| LA HAYE | 10-09-1990 | BRENNAN J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)